# EUROPEAN PATENT SPECIFICATION

(11) **EP 3 225 277 B1**
(45) Date of publication and mention of the grant of the patent: **20.02.2019**
(21) Application number: 17163663.2
(22) Date of filing: 29.03.2017
(51) Int. Cl.: A61M 37/00

(54) **MANUFACTURING METHOD OF SHEET HAVING NEEDLE-LIKE PROTRUDING PORTIONS**
VERFAHREN ZUR HERSTELLUNG EINER FOLIE MIT NADELFÖRMIGEN HERVORSTEHENDEN TEILEN
PROCÉDÉ DE FABRICATION DE FEUILLE PRÉSENTANT DES PARTIES SAILLANTES EN FORME D'AIGUILLES

(30) Priority: 30.03.2016 JP 2016068197
(43) Date of publication of application: 04.10.2017
(73) Proprietor: Fujifilm Corporation, Minato-ku Tokyo 106-8620 (JP)
(72) Inventor: OKANO, Keio, Kanagawa, 258-8538 (JP); KATAGIRI, Yoshinobu, Kanagawa, 258-8538 (JP)
(74) Representative: Klunker IP Patentanwälte PartG mbB

(56) References cited:
- EP-A1- 2 283 809
- EP-A1- 2 921 202
- JP-A- 2009 241 358
- JP-A- 2015 100 659
- US-A1- 2014 034 838

## Description

### BACKGROUND OF THE INVENTION

### Field of the Invention

The present invention relates to a manufacturing method of a sheet having needle-like protruding portions, and relates in particular to a method of manufacturing a sheet having needle-like protruding portions by shape transfer using a mold on which needle-like recessed portions are formed.

### Description of the Related Art

As a method of injecting medicine or the like from a biological surface, that is, the skin or mucous membrane or the like, a method of injecting medicine by using a transdermal absorption sheet on which needle-like protruding portions (hereinafter, referred also as "fine needles" or "microneedles") of a high aspect ratio containing a drug are formed and inserting the fine needles into the skin has been performed. For use as the transdermal absorption sheet, the drug needs to be mixed in; however, since many drugs are expensive, the drug needs to be contained intensively in the fine needles.

As a method of manufacturing the transdermal absorption sheet, a method is known in which a polymer solution or the like is poured into a mold where needle-like recessed portions that are inverted shapes of the needle-like protruding portions are formed to transfer the shapes. For example, there is a method of using a mold of a microneedle sheet in which through-holes passing through a base material are perforated at bottoms of the recessed portions, applying a solution in which a medicine is diluted first on the mold, scraping off excess solution with a squeegee or the like, drying the chemical solution and then applying a needle raw material.

In addition, there is a manufacturing method of a sheet in which fine needle-like protrusion formulations are accumulated in one step. In the method, the sheet is highly accurately manufactured by drying and curing a thick liquid formed of a mixture of a target material and a base material while filling the liquid to a flexible substrate with centrifugal force.

In microneedle formation using the shape transfer with a needle-like recessed intaglio, regardless of whether the chemical solution is contained or not, the polymer solution needs to be applied onto the needle-like recessed intaglio by some method. For example, Japanese Patent Application Laid-Open No. 2011-078617 describes a manufacturing method of a microneedle sheet, in which a raw material is injected to recessed portions of a stamper. International Publication No. WO2014/077242 (≙ EP2921202) describes a manufacturing method of a transdermal absorption sheet including a process of filling a solution in needle-like recessed portions of a mold in a state that a nozzle and a mold front surface are brought into contact, and relatively moving the nozzle and the mold in the state that the nozzle and the mold are brought into contact with each other.

### SUMMARY OF THE INVENTION

In the method described in Japanese Patent Application Laid-Open No. 2011-078617, since a squeegee is pressed against a mold when performing scraping-off with the squeegee, there is a problem that a volume of recessed portions of the stamper changes and a filling amount changes. Also, in International Publication No. WO2014/077242, similarly, while a chemical solution is efficiently filled by pressing a solution supply portion to a mold and performing discharge of only a required amount to needle-like recessed portions while preventing drying, since the chemical solution is filled in the needle-like recessed portions of the mold squashed by the solution supply portion, there is the problem that the volume of the needle-like recessed portions changes and the filling amount changes due to factors such as a mold crush angle, a pressing displacement amount and thickness variation. Therefore, efficient operations cannot be performed.

The present invention is implemented in consideration of such a situation, and aims to provide a manufacturing method of a sheet having needle-like protruding portions, capable of preventing deformation of a mold when a chemical solution is filled and improving filling accuracy by optimizing hardness of the mold to be used.

In order to achieve the object, the present invention provides a manufacturing method of a sheet having needle-like protruding portions, including: the features of claim 1 or claim 2.

According to the present invention, since the average value of the Young's modulus at the part within 40 µm from the front surface of the mold is in the above-described range, in the filling process and the moving process, even when the nozzle distal end portion is pressed against the front surface of the mold, it is possible to prevent the mold from being deformed and the volume of the needle-like recessed portions from being deformed. In addition, by setting an upper limit of the average value of the Young's modulus at the part within 40 µm from the front surface of the mold to be 100 MPa or lower, the nozzle distal end portion can be pressed into the mold front surface by a surface roughness amount so that a clearance between the nozzle distal end portion and the mold front surface can be eliminated, thereby the solution can be accurately filled in the needle-like recessed portions. Note that, in the present invention, the front surface of the mold refers to a surface where the needle-like recessed portions are formed. In the case that through-holes are provided on distal ends of the needle-like recessed portions, the front surface is a surface on a side where an opening is wide.

According to the present invention, since the average value of the Young's modulus at the part within 40 µm from the front surface of the mold is in the above-described range, in the moving process, even when the blade is pressed against the front surface of the mold, it is possible to prevent the mold from being deformed and the volume of the needle-like recessed portions from being deformed. In addition, by setting the upper limit of the average value of the Young's modulus at the part within 40 µm from the front surface of the mold to be 100 MPa or lower, the blade can be pressed into the mold front surface by a surface roughness amount so that a clearance between the blade and the mold front surface can be eliminated, thereby the solution can be accurately filled in the needle-like recessed portions.

As a hardness distribution in the thickness direction of the mold, an average value of a Young's modulus at a part over 40 µm from the front surface of the mold is lower than the average value of the Young's modulus at the part within 40 µm from the front surface of the mold.

According to this aspect, since the average value of the Young's modulus on a distal end side of the needle-like recessed portions of the mold is turned low, when the sheet having the needle-like protruding portions is released from the mold, it is possible to prevent the needle-like protruding portions from being damaged such as a scratch or a break of the needle-like protruding portions of the formed sheet.

In another aspect of the present invention, it is preferable that an average value of a Young's modulus in an entire thickness direction of the mold is 1.9 MPa or higher and 100 MPa or lower.

According to the aspect, by setting the Young's modulus in the entire thickness direction of the mold to the above-described range, a shape change in the distal end of the needle-like recessed portion can be suppressed, thereby filling can be accurately performed.

In another aspect of the present invention, it is preferable that the mold is formed of silicone resin.

According to this aspect, even in the case that the sheet having the needle-like protruding portions to be formed is a pharmaceutical product, the sheet having the needle-like protruding portions can be manufactured while maintaining safety. In addition, since the silicone resin is durable against pressure transfer, the number of uses (times of uses) of the mold can be increased, and a manufacturing cost can be lowered.

According to the manufacturing method of the sheet having needle-like protruding portions of the present invention, the variation in a crush amount of the needle-like recessed portions due to a pressing amount of the nozzle distal end portion or the blade and the thickness variation among the molds can be reduced when the solution is filled in the needle-like recessed portions. Thus, filling accuracy can be improved.

### BRIEF DESCRIPTION OF THE DRAWINGS

Fig. 1 is a perspective view of a fine needle (needle-like protruding portion) in a pyramid shape of a transdermal absorption sheet;
Fig. 2 is a sectional view of the fine needle (needle-like protruding portion) in the pyramid shape of the transdermal absorption sheet;

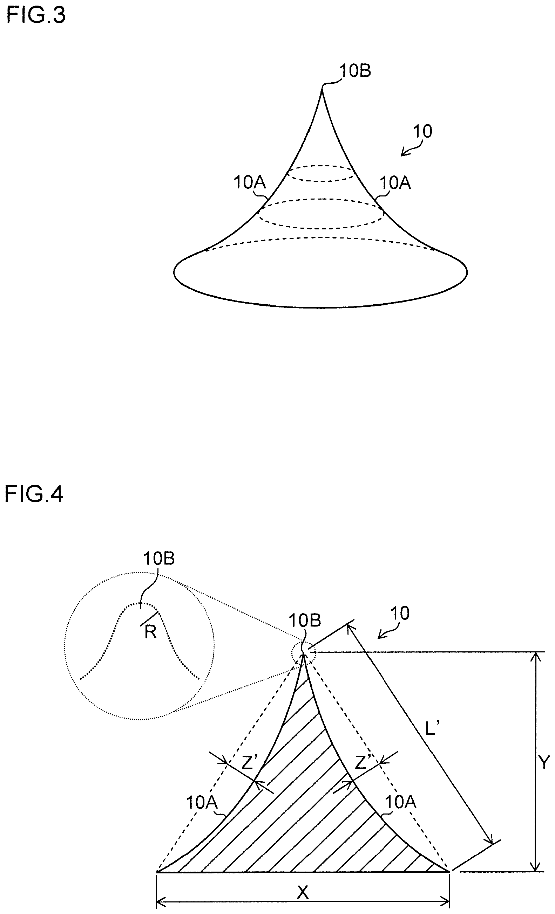

Fig. 3 is a perspective view of a fine needle (needle-like protruding portion) in a conical shape of the transdermal absorption sheet;
Fig. 4 is a sectional view of the fine needle (needle-like protruding portion) in the conical shape of the transdermal absorption sheet;
Fig. 5 is a diagram illustrating a process of a manufacturing method of a mold;
Fig. 6 is a diagram illustrating a process of the manufacturing method of the mold;
Fig. 7 is a diagram illustrating a process of the manufacturing method of the mold;
Fig. 8 is a sectional view illustrating another aspect of the mold;
Fig. 9 is a sectional view illustrating an aspect of a mold complex;
Fig. 10 is a schematic diagram illustrating a process of filling a drug-containing solution in the mold;
Fig. 11 is a schematic diagram illustrating the process of filling the drug-containing solution in the mold;
Fig. 12 is a schematic diagram illustrating the process of filling the drug-containing solution in the mold;
Fig. 13 is a perspective view illustrating a distal end portion of a nozzle;
Fig. 14 is a perspective view illustrating a distal end portion of another nozzle;
Fig. 15 is a partial enlarged view of a distal end of the nozzle and the mold during filling;
Fig. 16 is a partial enlarged view of the distal end of the nozzle and the mold during moving;
Fig. 17 is an explanatory drawing illustrating a relation between pressure reduction inside the nozzle and supply of the drug-containing solution;
Fig. 18 is an explanatory drawing illustrating another embodiment of a process of filling the drug-containing solution to the mold;
Fig. 19 is an explanatory drawing illustrating another embodiment of the process of filling the drug-containing solution to the mold;
Fig. 20 is an explanatory drawing illustrating another embodiment of the process of filling the drug-containing solution to the mold;
Fig. 21 is an explanatory drawing illustrating a formation process of a polymer sheet;
Fig. 22 is an explanatory drawing illustrating the formation process of the polymer sheet;
Fig. 23 is an explanatory drawing illustrating the formation process of the polymer sheet;
Fig. 24 is an explanatory drawing illustrating another formation process of the polymer sheet;
Fig. 25 is an explanatory drawing illustrating another formation process of the polymer sheet;
Fig. 26 is an explanatory drawing illustrating another formation process of the polymer sheet;
Fig. 27 is an explanatory drawing illustrating another formation process of the polymer sheet;
Fig. 28 is an explanatory drawing illustrating still another formation process of the polymer sheet;
Fig. 29 is an explanatory drawing illustrating still another formation process of the polymer sheet;
Fig. 30 is an explanatory drawing illustrating still another formation process of the polymer sheet;
Fig. 31 is an explanatory drawing illustrating still another formation process of the polymer sheet;
Fig. 32 is an explanatory drawing illustrating a release process;
Fig. 33 is an explanatory drawing illustrating another release process;
Fig. 34 is a sectional view of the transdermal absorption sheet;
Fig. 35 is a plan view and a side view of an original plate; and
Fig. 36 is a graph illustrating a variation of a filling amount to a Young's modulus of the mold.

### DETAILED DESCRIPTION OF THE PREFERRED EMBODIMENTS

Hereinafter, according to the attached drawings, a manufacturing method of a sheet having needle-like protruding portions relating to the present invention is described. Note that, in the present specification, "-" is used to mean that numerical values described before and after the values are included as a lower limit value and an upper limit value. In addition, while the description is given using "a transdermal absorption sheet on which fine needles (needle-like protruding portions) with a high aspect ratio containing a drug are formed" as one example of "the sheet having the needle-like protruding portions"; however, the present invention is not limited to the sheet, and can be used for any sheet as long as it is the sheet having the needle-like protruding portions.

### (Transdermal absorption sheet)

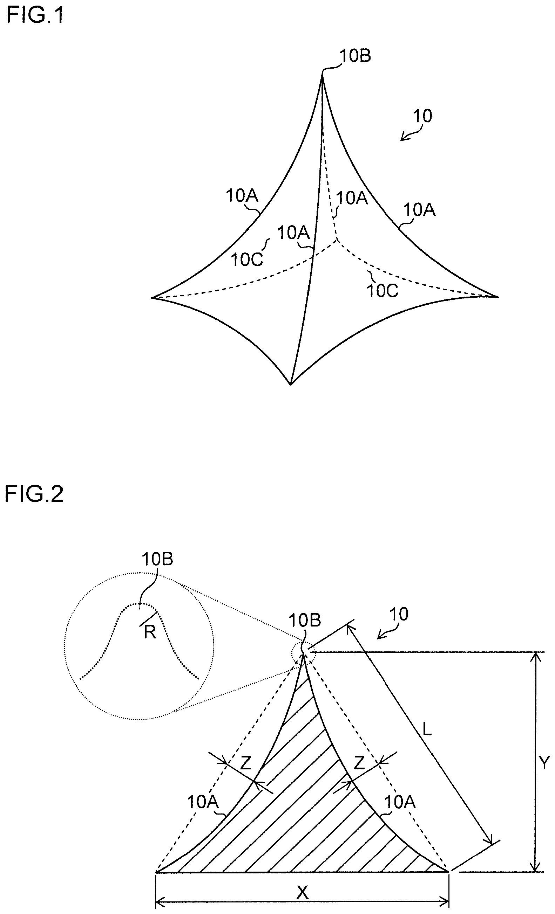

The needle-like protruding portions (called also fine needles or microneedles) of the sheet manufactured by the manufacturing method of the sheet having the needle-like protruding portions in an embodiment of the present invention are described. Fig. 1 is a perspective view of the fine needle (needle-like protruding portion) in a pyramid shape of the transdermal absorption sheet, and Fig. 2 is a sectional view thereof. In the embodiment, an example of the needle-like protruding portion in a quadrangular pyramid shape is described; however, the shape is not limited to the quadrangular pyramid shape.

As illustrated in Figs. 1 and 2, for the shape of a fine needle (needle-like protruding portion) 10 formed on the transdermal absorption sheet, in order to stick the fine needle 10 to a skin surface to a depth of several 100 µm, it is preferable that (1) a distal end is sufficiently pointed and a diameter of the needle to enter into the skin is sufficiently small (an aspect ratio of length/diameter is high), and (2) the needle has sufficient strength (the needle does not bend or the like).

Therefore, although a thin and sharp shape is needed in order to meet a requirement (1), it is opposed to (2). An excessively thin needle is bent at a distal end or a root, and an excessively thick needle fails to be stuck. Thus, as illustrated in Fig. 1, it is preferable that a ridge line 10A of the fine needle 10 is in a shape of being curved toward an inner side of the fine needle. By attaining such a shape, while the distal end is sufficiently sharpened, the root is widened and the needle becomes hard to break. In addition, it is preferable that the ridge lines 10A and 10A of the fine needle in the quadrangular pyramid shape are extended outward from a quadrangular pyramid surface 10C between the ridge lines.

For the shape of the fine needle 10, it is preferable that one side X of a bottom surface is in a range of 0.1 µm or more and 1000 µm or less, and a height is 0.3 µm or more and 3000 µm or less. More preferably, one side X is in a range of 10 µm or more and 400 µm or less, and the height is 30 µm or more and 1200 µm or less.

Then, it is preferable that, when a length of a line segment connecting a start point and an end point of the ridge line is defined as L, a maximum depth Z of a curve of the ridge line 10A is 0.04 × L or more and 0.2 × L or less. In addition, a curvature radius R of a fine needle distal end 10B indicating sharpness of the fine needle 10 is preferably 20 µm or less, and more preferably 15 µm or less.

Note that, while Figs. 1 and 2 illustrate the fine needle 10 in the quadrangular pyramid shape, it is preferable that the fine needles in the conical shape and in other pyramid shapes such as a triangular pyramid are in a similar size. Note that, in the case of the conical shape, a diameter X of the bottom surface is preferably in the range of 0.1 µm or more and 1000 µm or less, and more preferably in the range of 50 µm or more and 500 µm or less. In addition, it is preferable that, when a length of a line segment connecting a start point and an end point of a generating line of a conical surface is defined as L', a maximum depth Z' of the curve of the conical surface is 0.04 × L' or more and 0.2 × L' or less.

As described above, the transdermal absorption sheet is a protruding portion array in which the fine needles are arrayed in a two-dimensional array. In order to make it easy to be stuck to the skin surface, it is important to make the fine needle distal end 10B sharp and sufficiently pointed. It is preferable that a curvature radius R of the fine needle distal end 10B is 20 µm or less. In order to form the fine needles 10 each having a distal end with the curvature radius R of 20 µm or less, it is an important point whether a solution of a polymer resin can be injected to the distal ends (bottoms) of needle-like recessed portions which are an inverted shape of the protruding array formed on the mold to perform precise transfer.

In addition, for the transdermal absorption sheet, a drug needs to be mixed in; however, since many drugs are expensive, it is important in terms of costs that the drug is contained intensively at fine needle portions and is accurately filled.

### [Manufacturing method of transdermal absorption sheet]

Next, the manufacturing method of the sheet having the needle-like protruding portions in an embodiment of the present invention is described with the transdermal absorption sheet provided with the fine needles as an example.

### (Fabrication of mold)

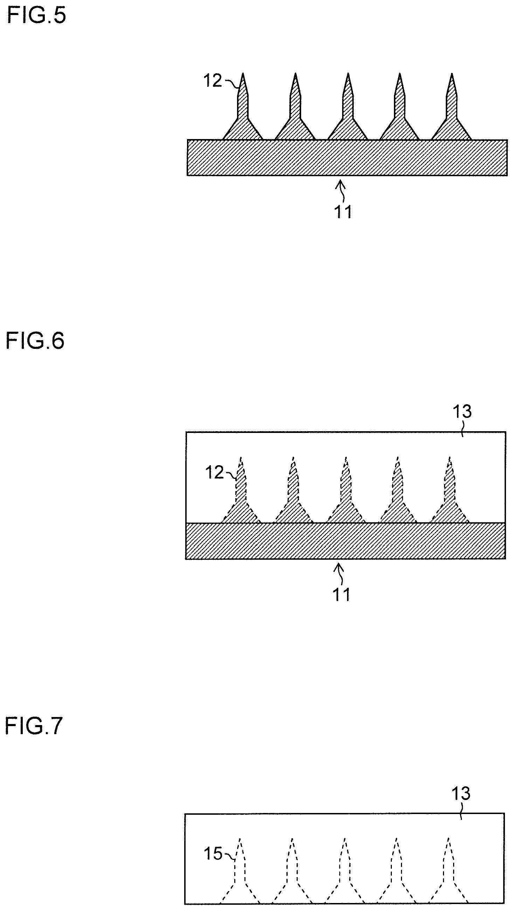

Figs. 5 to 7 are drawings illustrating processes of fabricating the mold.

As illustrated in Fig. 5, an original plate for fabricating the mold for manufacturing the transdermal absorption sheet is fabricated first.

There are two kinds of fabrication methods of an original plate 11. In the first method, after applying photoresist onto a Si substrate, exposure and development are performed. Then, by performing etching such as RIE (reactive ion etching) or the like, an array of conical shape portions (needle-like protruding portions) 12 is fabricated on a front surface of the original plate 11. Note that, when performing the etching such as the RIE so as to form the conical shape portions on the front surface of the original plate 11, by performing the etching from an oblique direction while rotating the Si substrate, a conical shape can be formed.

The second method is a method of forming the array of the needle-like structure portions 12 of a quadrangular pyramid or the like on the front surface of the original plate 11 by machining using a cutting tool such as a diamond tool.

Next, the mold is fabricated. Specifically, as illustrated in Fig. 6, a mold 13 is fabricated from the original plate 11. The original plate 11 has a conical shape or a pyramid shape (a quadrangular pyramid, for example) with a sharp distal end. The following methods are conceivable as a method which can inexpensively manufacture the mold 13 while accurately transferring the shape of the original plate 11 to the mold 13 and releasing the mold 13 from the original plate 11.

The first method includes the steps of: pouring a silicone resin which is prepared by adding a curing agent to PDMS (polydimethylsiloxane: Sylgard 184 made by Dow Corning Corp., Sylgard is a registered trademark), to the original plate 11; curing the resin by heat treatment at 100°C; and then releasing it from the original plate 11. The second method includes the steps of: pouring a UV (ultraviolet) curing resin which is curable by being irradiated with ultraviolet rays to the original plate 11; irradiating the resin with the ultraviolet rays in a nitrogen atmosphere; and then releasing it from the original plate 11. The third method includes the steps of: pouring a solution prepared by dissolving plastic resin such as polystyrene or PMMA (polymethyl methacrylate) in an organic solvent to the original plate 11 to which a release agent has been applied; curing the solution by evaporating the organic solvent by drying, and then releasing it from the original plate 11.

In this way, the mold 13 on which needle-like recessed portions 15 having the inverted shape of the conical shapes or the pyramid shapes on the original plate 11 are two-dimensionally arrayed is fabricated. The mold 13 fabricated in this way is illustrated in Fig. 7. Note that the mold 13 can be easily fabricated any number of times by any of the above-described methods.

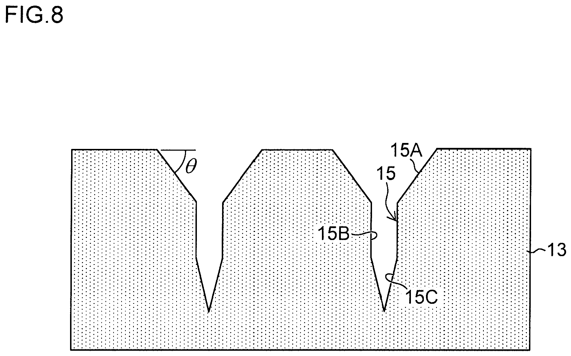

Fig. 8 illustrates an aspect of another preferable mold 13. The needle-like recessed portion 15 includes a tapered entrance portion 15A narrowed in a depth direction from the front surface of the mold 13, an intermediate recessed portion 15B having a fixed width in the depth direction, and a distal end recessed portion 15C tapered in the depth direction. A taper angle 0 of the taper is preferably, in a range of 10° to 20°. The entrance portion 15A having a tapered shape enables to easily fill a polymer solution in the needle-like recessed portion 15.

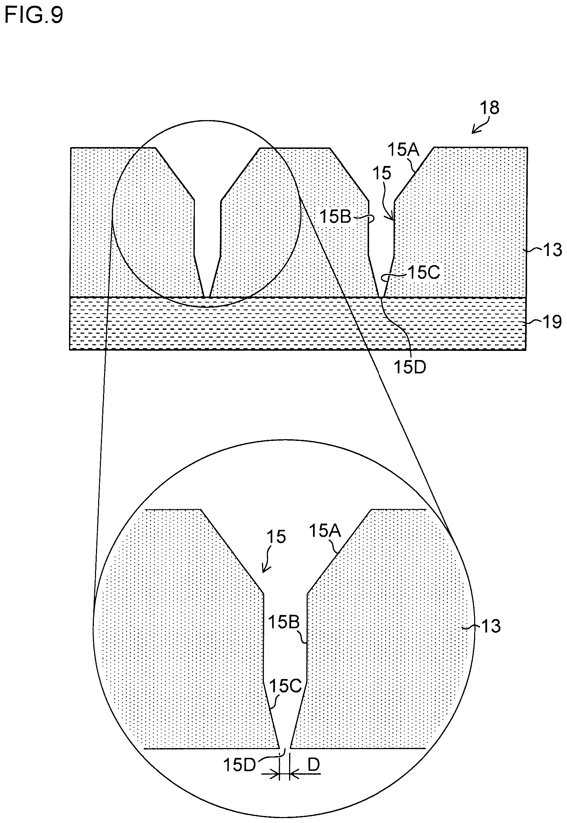

Fig. 9 illustrates an aspect of a mold complex 18 which is more preferable when performing the manufacturing method of the transdermal absorption sheet.

As illustrated in Fig. 9, the mold complex 18 includes: the mold 13 with an air vent hole 15D formed at the distal end (bottom) of the needle-like recessed portion 15; and a gas permeable sheet 19 which is stuck to a back surface of the mold 13 and formed of a material that permeates a gas but does not permeate a liquid. The air vent hole 15D is formed as a through-hole that passes through the back surface of the mold 13. Here, the back surface of the mold 13 refers to a surface on a side where the air vent hole 15D is formed. Thus, the distal end of the needle-like recessed portion 15 is communicated with the atmosphere through the air vent hole 15D and the gas permeable sheet 19.

By using such a mold complex 18, a transdermal absorption material solution filled in the needle-like recessed portions 15 is not permeated, and only the air existing in the needle-like recessed portions 15 can be discharged from the needle-like recessed portions 15. Thus, a transfer property (transferability) of transferring the shape of the needle-like recessed portions 15 to a transdermal absorption material is improved, and a sharper fine needle 10 can be formed.

As a diameter D (diameter) of the air vent hole 15D, the range of 1 µm to 50 µm is preferable. A role as an air vent hole cannot be sufficiently achieved when it is shorter than 1 µm, and sharpness of the distal end portion of the molded fine needle 10 is impaired when it exceeds 50 µm.

As the gas permeable sheet 19 formed of the material that permeates a gas but does not permeate a solution, latex (Asahi Kasei Chemicals Corp.), for example, can be suitably used.

The material to be used for the mold 13, in terms of a hardness distribution in a thickness direction of the mold 13, has a hardness of 1.9 MPa or higher and 100 MPa or lower as an average value of a Young's modulus at a part within 40 µm at least from a front surface side of the mold 13. When the mold 13 has the Young's modulus in the above-described range, the shape change of the mold 13 can be suppressed, and the material for forming the transdermal absorption sheet (also referred to as "transdermal absorption material", hereinafter) can be accurately filled in the needle-like recessed portions 15 of the mold 13.

The solution is filled into the mold 13 by using a nozzle (slit nozzle) or a blade. At that time, in order not to leak the solution on a back side in the movement direction of the nozzle, it is required that the nozzle or the blade and the mold are brought into close contact with each other. Since a the mold has a variation in film thickness a base part for mounting the mold during filling of the solution has minute roughness, the mold has a variation in height and a surface roughness, each of which is 10 µm to 20 µm. On the other hand, the nozzle or the blade also has the roughness of 10 µm to 20 µm. Therefore, in order to bring the mold into close contact with the nozzle or the blade, the nozzle or the blade needs to be pushed into the mold by at least about 40 µm so as to deform the mold. Thus, each roughness is absorbed and the solution can be filled without leakage.

When the Young's modulus of the front surface of the mold is low, crush of the front surface of the mold due to local thickness irregularity of the mold greatly affects the crush of the needle-like recessed portions. By configuring the front surface side of the mold so as to have a Young's modulus in the above-described range, even when the mold has a local thickness irregularity, the thickness irregularity can be absorbed by the entire surface of the mold, and local deformation of the needle-like recessed portions can be mitigated.

Note that, when molding the mold formed of multiple layers, the average value of the Young's modulus refers to a value obtained by separately measuring a Young's modulus for each of the layers, and calculating a weighting average value of the Young's modulus for each of the layers by using the film thickness of each layer. For example, in the case where the mold is formed of: the material having a Young's modulus of "a" MPa and a thickness of A µm; the material having a Young's modulus of "b" MPa and a thickness of B µm; and the material having Young's modulus of "c" MPa and a thickness of C µm, the average value of the Young's modulus is expressed by "(a × A + b × B + c × C) ÷ (A + B + C)." The Young's modulus of each layer of the mold is measured in the following manner. First, a single mold is fabricated with a material of each layer under the same condition separately from the mold to perform filling. For each mold, the Young's modulus is measured and calculated based on a load when displacement of 40 µm is given in a compression test for a side part of the needle-like recessed portions of the mold before filling the transdermal absorption material, using a micro-hardness meter HM2000 made by FISCHER INSTRUMENTS K.K. and a Berkovich indenter.

When filling the transdermal absorption material to the mold 13, in the state that the nozzle distal end portion or the blade is pressed against the front surface of the mold 13 so as to be brought into contact with the front surface of the mold 13, the transdermal absorption material is filled, and the mold 13 and a solution supply device (the nozzle or the blade) are moved, although the filing process is described later. If the Young's modulus of the front surface of the mold 13 is low, when the solution supply device is pressed, the mold 13 is deformed so that a volume of the needle-like recessed portions 15 is changed and the variation of the filling amount in the needle-like recessed portions 15 is generated. In addition, even when the Young's modulus of the mold 13 is too high, when the solution supply device is pressed, the solution supply device cannot be sufficiently pushed in the mold 13 so that a clearance is generated between the mold 13 and the solution supply device. When the transdermal absorption material enters the clearance, efficiency is degraded in terms of manufacturing and filling accuracy is deteriorated. By setting the Young's modulus of the mold 13 to be in the above-described range, the shape change of the needle-like recessed portions 15 of the mold 13 can be suppressed, and filling can be performed without generating a clearance between the solution supply device and the mold 13.

The average value of the Young's modulus at the part within 40 µm from the front surface side of the mold is preferably 1.9 MPa or higher, more preferably 2.5 MPa or higher, and even more preferably 3 MPa or higher. Also, it is preferably 100 MPa or lower, more preferably 50 MPa or lower, and even more preferably 35 MPa or lower.

As such a material, specifically, silicone resin or the like can be used. Since the silicone resin is durable against transfer by repetitive pressurization and detachability with a material is excellent, it can be suitably used. In addition, in the case that the manufactured sheet such as the transdermal absorption sheet is a pharmaceutical product, safety can be maintained. In addition, by using a material with high gas permeability, when filling the transdermal absorption material, the air existing in the recessed portions of the mold can be discharged from a mold side so that the needle-like protruding portions can be accurately manufactured. Regarding the high gas permeability, oxygen permeability representative of the gas permeability is preferably greater than 1 x 10⁻¹² (mL/s·m²·Pa), and further preferably greater than 1 × 10⁻¹⁰ (mL/s·m²·Pa).

For the Young's modulus of the mold 13, the average value of the Young's modulus in the entire thickness direction can be 1.9 MPa or higher and 100 MPa or lower. By setting the Young's modulus in the entire thickness direction of the mold 13 to be in the above-described range, the shape change of the part of the distal end of the needle-like recessed portions 15 can be suppressed, and filling can be accurately performed.

In addition, in the thickness direction of the mold 13, the average value of the Young's modulus at the part over 40 µm from the front surface can be lower than the average value of the Young's modulus at the part within 40 µm. By raising the Young's modulus on the front surface side of the mold 13 and lowering the Young's modulus on a back surface side, the volume change of the needle-like recessed portions 15 of the mold 13 can be suppressed, and when releasing the manufactured transdermal absorption sheet, the breakage of the needle-like protruding portions can be prevented.

Regarding the fabrication method of the mold 13 having a different (varying) Young's modulus in the thickness direction, it can be fabricated by changing the resin to be poured into the original plate 11. For example, the mold of the different Young's modulus may be manufactured in the following manner. After pouring the resin of the high Young's modulus (the resin to be the front surface side of the mold 13), curing and drying of the resin is performed. Then, the resin of the low Young's modulus (the resin to be the back surface side of the mold 13) is poured, and then, curing and drying of the resin is performed. In addition, the mold may also be manufactured by after pouring the resin of the low Young's modulus, then pouring the resin of the high Young's modulus, and simultaneously curing and drying both resins.

### (Polymer solution)

The polymer solution which is a solution of the polymer resin to be the material of the transdermal absorption sheet used in the embodiment is described.

As the material of a resin polymer used for the polymer solution, it is preferable to use a resin with biocompatibility. As such a resin, it is preferable to use saccharides such as glucose, maltose, pullulan, sodium chondroitin sulfate, sodium hyaluronate, hydroxypropyl cellulose or hydroxyethyl starch, protein such as gelatin, or a biodegradable polymer such as polylactic acid or lactic acid/glycolic acid copolymer. Among them, a gelatin-based material is adhesive with many release plates and has high gel strength as a material to be gelled, so that it can be closely attached with the release plate and a polymer sheet can be released using the release plate from the mold in a release process to be described later, and thus, it can be suitably utilized. A concentration is different depending on the material; however, the concentration that 10% to 50% of the resin polymer is contained in the solution is preferable. In addition, a solvent used in dissolution may be the one with volatility even when it is other than hot water, and methyl ethyl ketone (MEK) or alcohol or the like may be used. In the solution of the polymer resin, the drug (medicine) to be supplied into the body according to a use can be dissolved together.

As a preparation method of the polymer solution, in the case of using a watersoluble high polymer (gelatin or the like), it can be manufactured by dissolving watersoluble powder in water and then adding the medicine after dissolution. In the case that it is hard to be dissolved in water, heating may be performed for dissolution. While a temperature can be appropriately selected depending on a kind of a high polymer material, it is preferable to perform heating at the temperature of about 60°C or lower. In addition, in the case of using a high polymer (maltose or the like) melts with heat, the polymer solution can be manufactured by heating a raw material and the medicine to melt the row material. Regarding a heating temperature, it is preferable to perform heating at the temperature at which the raw material melts, and it is about 150°C specifically.

In the embodiment, the polymer solution containing the drug is called a drug-containing solution, and the polymer solution not containing the drug is called a non-drug-containing solution, as needed.

Viscosity of the solution of the polymer resin is preferably 100 Pa·s or lower for the drug-containing solution, and more preferably 10 Pa·s or lower. For the non-drug-containing solution, it is preferably 2000 Pa·s or lower, and more preferably 1000 Pa·s or lower. By appropriately adjusting the viscosity of the solution of the polymer resin, it becomes easy to inject the solution to the recessed portions of the mold.

### <Manufacture of transdermal absorption sheet>

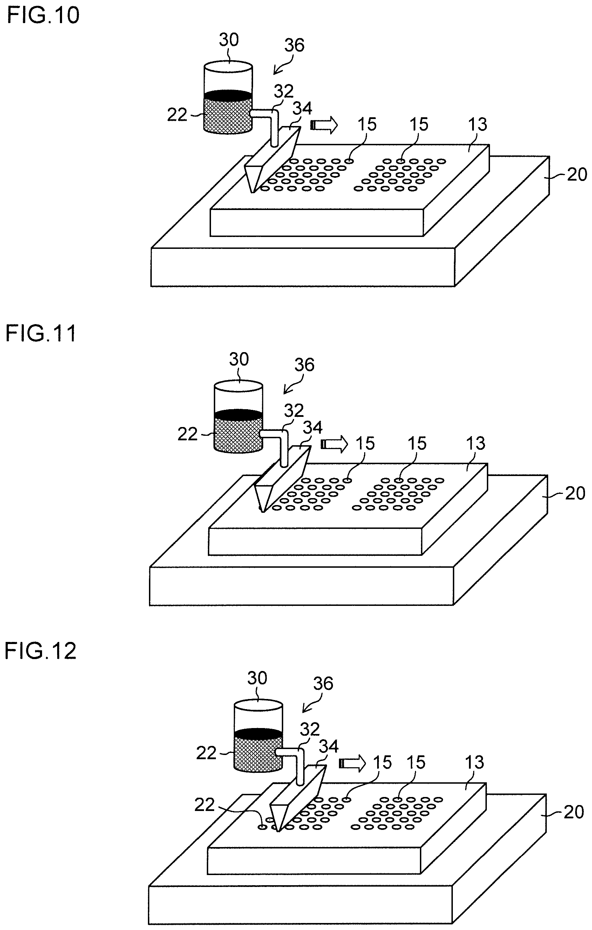

The manufacturing method of the transdermal absorption sheet using the mold 13 manufactured as described above is described. In Figs. 10 to 12, the mold 13 having the two-dimensionally arrayed needle-like recessed portions 15 is placed on a base 20. In the mold 13, two sets of the plurality of needle-like recessed portions 15 in a 5×5 two-dimensional array are formed. A solution supply device 36 including a tank 30 that stores a drug-containing solution 22, piping 32 connected to the tank, and a nozzle 34 connected to the distal end of the piping 32 is prepared (a device preparation process).

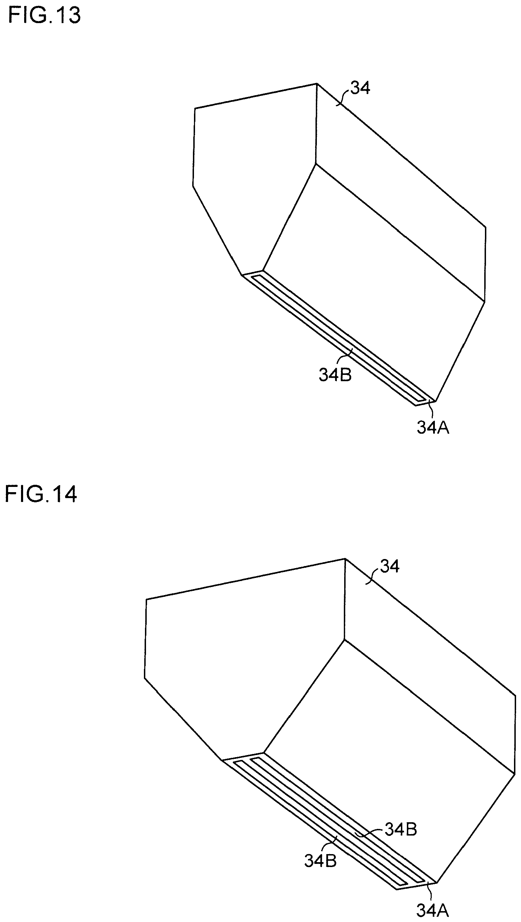

Fig. 13 illustrates a schematic perspective view of the distal end portion of the nozzle. As illustrated in Fig. 13, on the distal end of the nozzle 34, a lip portion 34A which is a flat surface and an opening 34B formed into a slit shape are provided. With the opening 34B having the slit-shape, for example, the drug-containing solution 22 can be simultaneously filled into the plurality of needle-like recessed portions 15 constituting one column. The size (length and width) of the opening 34B is appropriately selected according to the number of the needle-like recessed portions 15 to be filled at one time.

By making the length of the opening 34B longer, the drug-containing solution 22 can be filled into more needle-like recessed portions 15 at one time. Thus, productivity can be improved.

Fig. 14 illustrates a schematic perspective view of a distal end portion of another nozzle. As illustrated in Fig. 14, the lip portion 34A at the distal end of the nozzle 34 is provided with two openings 34B each of which is formed in a slit shape. With the two openings 34B, for example, the drug-containing solution 22 can be simultaneously filled into the plurality of needle-like recessed portions 15 constituting two columns.

As the material used for the nozzle 34, an elastic material or a metallic material can be used. The examples include Teflon (registered trademark), stainless steel, and titanium.

A filling process is described with reference to Fig. 11. As illustrated in Fig. 11, the position of the opening 34B of the nozzle 34 is adjusted onto the needle-like recessed portions 15. The lip portion 34A of the nozzle 34 and the front surface of the mold 13 are brought into contact with each other. The drug-containing solution 22 is supplied from the solution supply device 36 to the mold 13, and the drug-containing solution 22 is filled into the needle-like recessed portions 15 from the opening 34B of the nozzle 34. In the embodiment, the drug-containing solution 22 is simultaneously filled into the plurality of needle-like recessed portions 15 configuring one column. However, without being limited thereto, the needle-like recessed portions 15 can be filled one by one. In addition, by using the nozzle 34 illustrated in Fig. 14 the drug-containing solution 22 can be simultaneously filled into the plurality of needle-like recessed portions 15 configuring a plurality of columns, for every columns.

In the case that the mold 13 is configured by the material with the gas permeability, the drug-containing solution 22 can be sucked by performing suction from the back surface of the mold 13, and filling of the drug-containing solution 22 into the needle-like recessed portions 15 can be accelerated.

Following the filling process, as illustrated in Fig. 12, while bringing the lip portion 34A of the nozzle 34 and the front surface of the mold 13 into contact with each other, the solution supply device 36 is relatively moved in a direction perpendicular to a length direction of the opening 34B, and the nozzle 34 is moved to the needle-like recessed portions 15 not filled with the drug-containing solution 22. The position of the opening 34B of the nozzle 34 is adjusted onto the needle-like recessed portions 15. In the embodiment, an example of moving the nozzle 34 is described; however, the mold 13 may be moved.

Since moving is performed while the lip portion 34A of the nozzle 34 and the front surface of the mold 13 are brought into contact with each other, the nozzle 34 can scrape off the drug-containing solution 22 remaining on the front surface other than the needle-like recessed portions 15 of the mold 13. The drug-containing solution 22 can be prevented from remaining anywhere other than the needle-like recessed portions 15 of the mold 13.

By repeating the filling processing in Fig. 11 and a moving process in Fig. 12, the drug-containing solution 22 is filled into the 5×5 two-dimensionally arrayed needle-like recessed portions 15. When the drug-containing solution 22 is filled into the 5×5 two-dimensionally arrayed needle-like recessed portions 15, the solution supply device 36 is moved to the adjacent 5×5 two-dimensionally arrayed needle-like recessed portions 15, and the filling process in Fig. 11 and the moving process in Fig. 12 are repeated. The drug-containing solution 22 is filled also to the adjacent 5×5 two-dimensionally arrayed needle-like recessed portions 15.

For the filling process and the moving process described above, (1) an aspect may be such that the drug-containing solution 22 is filled into the needle-like recessed portions 15 while the nozzle 34 is moved, or (2) an aspect may be such that the nozzle 34 is tentatively made to stand still on the needle-like recessed portions 15 while the nozzle 34 is moved, the drug-containing solution 22 is filled, and the nozzle 34 is moved again after filling. Between the filling process and the moving process, the lip portion 34A of the nozzle 34 is in contact with the front surface of the mold 13.

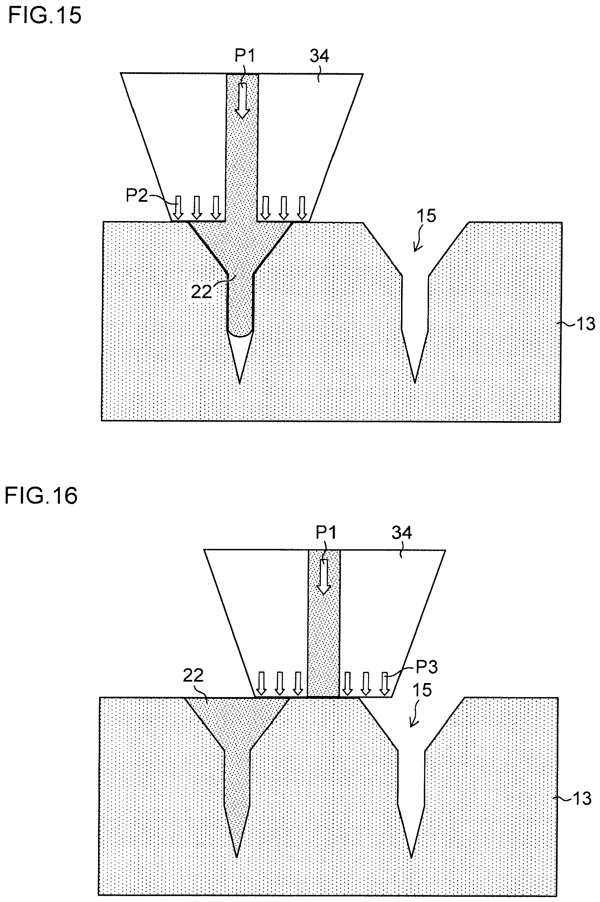

Fig. 15 is a partial enlarged view of the distal end of the nozzle 34 and the mold 13 while filling the drug-containing solution 22 into the needle-like recessed portions 15. As illustrated in Fig. 15, by applying pressurizing force P1 to the inside of the nozzle 34, the filling of the drug-containing solution 22 into the needle-like recessed portions 15 can be accelerated. Further, when filling the drug-containing solution 22 into the needle-like recessed portions 15, it is preferable to set a pressing force P2 which brings the nozzle 34 into contact with the front surface of the mold 13 to be equal to or greater than the pressurizing force P1 inside the nozzle 34. By attaining the pressing force P2 ≥ the pressurizing force PI, leakage of the drug-containing solution 22 from the needle-like recessed portions 15 to the front surface of the mold 13 can be suppressed.

Fig. 16 is a partial enlarged view of the distal end of the nozzle 34 and the mold 13 while moving the nozzle 34. When moving the nozzle 34 relatively to the mold 13, it is preferable to make pressing force P3 which brings the nozzle 34 into contact with the front surface of the mold 13 be smaller than the pressing force P2 which brings the nozzle 34 into contact with the front surface of the mold 13 during filling. It is to reduce damages to the mold 13 and suppress deformation due to compression of the mold 13.

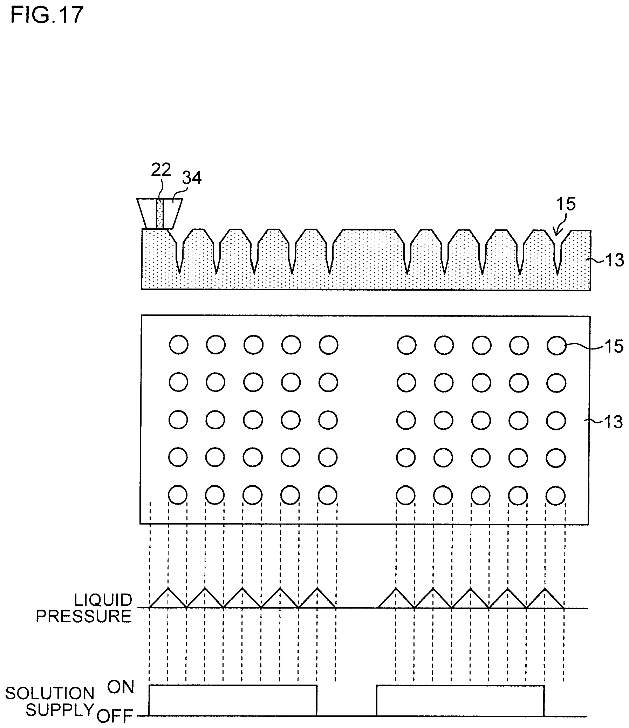

Fig. 17 is an explanatory drawing illustrating a relation between a liquid pressure inside the nozzle and supply of the drug-containing solution. As illustrated in Fig. 17, the supply of the drug-containing solution 22 is started before the nozzle 34 is positioned on the needle-like recessed portions 15. This enables to surely fill the drug-containing solution 22 into the needle-like recessed portions 15. Until the filling to the plurality of needle-like recessed portions 15 configured in 5×5 is completed, the drug-containing solution 22 is continuously supplied to the mold 13. The supply of the drug-containing solution 22 to the mold 13 is stopped before the nozzle 34 is positioned on the needle-like recessed portions 15 constituting the fifth column. This enables to prevent the drug-containing solution 22 from overflowing from the needle-like recessed portions 15. Regarding the liquid pressure inside the nozzle 34, after the supply of the drug-containing solution 22 is started, the liquid pressure becomes high in an area where the nozzle 34 is not positioned at the needle-like recessed portions 15. On the other hand, when the nozzle 34 is positioned on the needle-like recessed portions 15, the drug-containing solution 22 is filled into the needle-like recessed portions 15, and the liquid pressure inside the nozzle 34 becomes low. Fluctuation of the liquid pressure is repeated.

When the filling to the plurality of needle-like recessed portions 15 c is completed, the nozzle 34 is moved to the adjacent plurality of needle-like recessed portions 15 constituted of the 5×5 array. Regarding solution supply, when the nozzle 34 is moved to the adjacent plurality of needle-like recessed portions 15 constituted of the 5×5 array, it is preferable to stop the supply of the drug-containing solution 22. There is a distance from the needle-like recessed portions 15 constituting the fifth column to the needle-like recessed portions 15 constituting the next first column. When the drug-containing solution 22 is continuously supplied while the nozzle 34 is moved between them, the liquid pressure inside the nozzle 34 sometimes becomes too high. As a result, the drug-containing solution 22 sometimes overflows from the nozzle 34 to a part other than the needle-like recessed portions 15 of the mold 13. In order to suppress this problem, it is preferable to stop the supply of the drug-containing solution 22.

When the filling of the drug-containing solution 22 into the needle-like recessed portions 15 is completed, the process advances to a process of forming a polymer sheet which has the needle-like protruding portions on the front surface, and includes a drug-containing layer configured by the drug-containing solution 22 and a non-drug-containing layer configured by the non-drug-containing solution. The needle-like protruding portion has the inverted shape of the needle-like recessed portion.

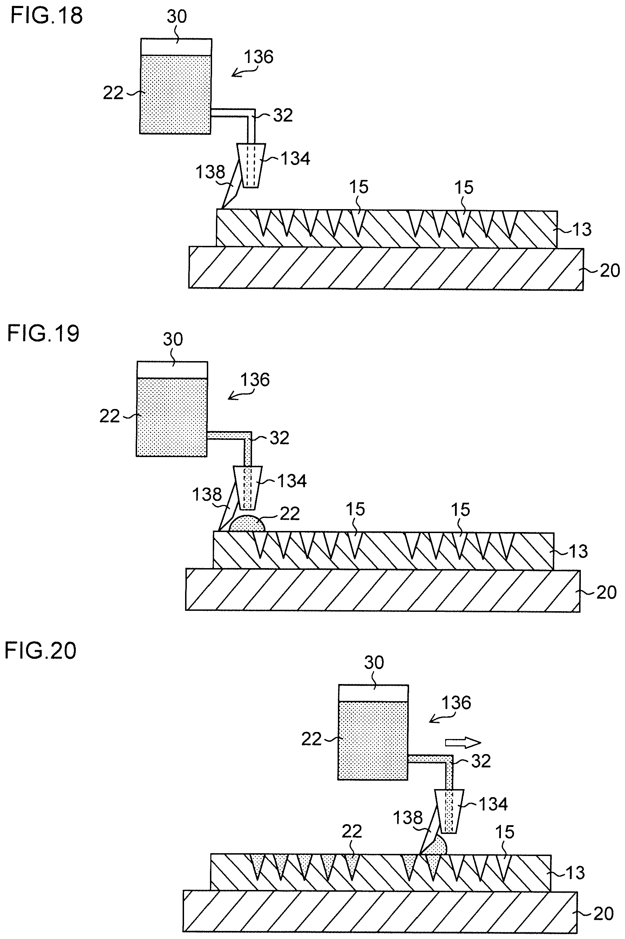

Next, another embodiment of the process of filling the drug-containing solution is described. Figs. 18 to 20 are drawings illustrating the process of filling the drug-containing solution 22 to the needle-like recessed portions 15 using a solution supply device 136 including a blade 138.

Also, in the solution supply device 136 including the blade 138, similarly to the case of pressing the nozzle distal end portion and filling the transdermal absorption material to the needle-like recessed portions illustrated in Figs. 10 to 12, the mold 13 having the two-dimensionally arrayed needle-like recessed portions 15 is placed on the base 20. On the mold 13, two sets of the 5×5 two-dimensionally arrayed needle-like recessed portions 15 are formed. The solution supply device 136 including the tank 30 that stores the drug-containing solution 22, the piping 32 connected to the tank 30, a nozzle 134 connected to the distal end of the piping 32, and the blade 138 is prepared.

Next, as illustrated in Fig. 19, the drug-containing solution 22 is supplied from the solution supply device 136 to the mold 13 (solution supply process). The nozzle 134 has an opening formed into a slit shape as illustrated in Fig. 13, and the drug-containing solution 22 can be supplied between the plurality of needle-like recessed portions 15 constituting one column and the blade 138. A supply method of the drug-containing solution 22 to the mold 13 is not limited to such a nozzle having the opening in the slit shape. Supply can also be performed by a dispenser or the like.

Next, as illustrated in Fig. 20, the solution supply device 136 is moved relative to the mold 13 while bringing the blade 138 and the front surface of the mold 13 into contact with each other, and the drug-containing solution 22 supplied onto the mold 13 is moved to the needle-like recessed portions 15 not filled with the drug-containing solution 22 with the blade 138 (moving process).

When moving the solution supply device 136, since it is moved while bringing the blade 138 and the front surface of the mold 13 into contact with each other, the blade 138 can scrape off the drug-containing solution 22 remaining on the front surface other than the needle-like recessed portions 15 of the mold 13. The drug-containing solution 22 can be prevented from remaining anywhere other than the needle-like recessed portions 15 of the mold 13. Note that, in the embodiment, the example in which the solution supply device 136 is moved is described; however, the mold 13 may be moved. In addition, the solution supply device 136 illustrated in Figs. 18 to 20 is integrated with the blade 138; however, they may be separately configured.

As a material used for the blade 138, the material similar to the material used for the nozzle 34 can be used, and the elastic material or the metallic material can be used. The examples include Teflon (registered trademark), stainless steel, and titanium.

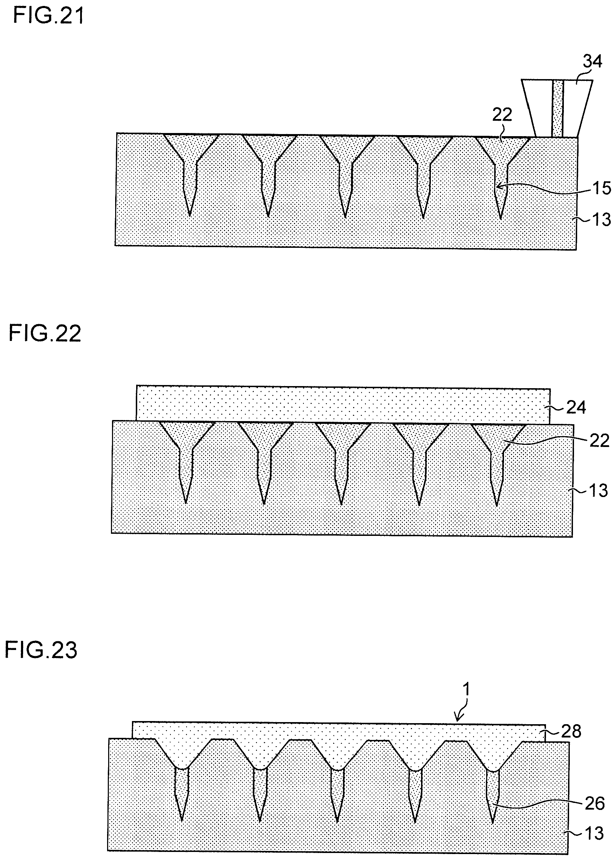

For the process of forming the polymer sheet, some aspects are described. A first aspect is described with reference to Figs. 21 to 23. As illustrated in Fig. 21, the drug-containing solution 22 is filled in the needle-like recessed portions 15 of the mold 13. As a filling method, filling may be performed by bringing the distal end portion of the nozzle into contact with the mold as illustrated in Figs. 10 to 12, or filling may be performed by bringing the blade into contact with the mold as illustrated in Figs. 18 to 20. Fig. 21 illustrates the process of filling the drug-containing solution 22 by bringing the distal end portion of the nozzle 34 into contact with the front surface of the mold 13. Next, as illustrated in Fig. 22, a non-drug-containing solution 24 is applied onto the drug-containing solution 22 by a dispenser or the like. In addition to coating by the dispenser, bar coating, spin coating and coating by a spray or the like can be adopted.

Next, as illustrated in Fig. 23, by drying and curing the drug-containing solution 22 and the non-drug-containing solution 24, a polymer sheet 1 composed of a drug-containing layer 26 and a non-drug-containing layer 28 is formed.

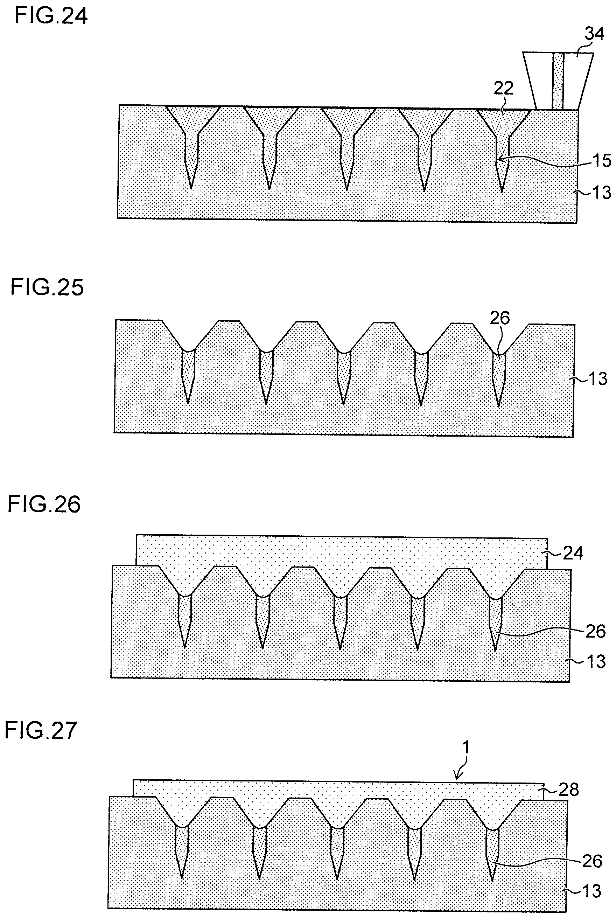

Next, a second aspect is described with reference to Figs. 24 to 27. As illustrated in Fig. 24, the drug-containing solution 22 is filled in the needle-like recessed portions 15 of the mold 13. As the filling method, similarly to the first aspect, filling may be performed by bringing the distal end portion of the nozzle 34 into contact with the mold 13, or filling may be performed by bringing the blade into contact with the mold 13. Next, as illustrated in Fig. 25, the drug-containing solution 22 is dried and cured to form the drug-containing layer 26 inside the needle-like recessed portions 15. When drying and curing the drug-containing solution 22, with reduced pressure suction from the back surface of the mold 13, the drug-containing solution 22 can be filled to the distal end of the needle-like recessed portions 15. Then, as illustrated in Fig. 26, the non-drug-containing solution 24 is applied onto the drug-containing layer 26 by the dispenser. In addition to coating by the dispenser, bar coating, spin coating and coating by a spray or the like can be adopted. Since the drug-containing layer 26 is cured, the drug inside the drug-containing layer 26 can be suppressed from spreading to the non-drug-containing solution 24.

Next, as illustrated in Fig. 27, by drying and curing the non-drug-containing solution 24, the polymer sheet 1 composed of the drug-containing layer 26 and the non-drug-containing layer 28 is formed.

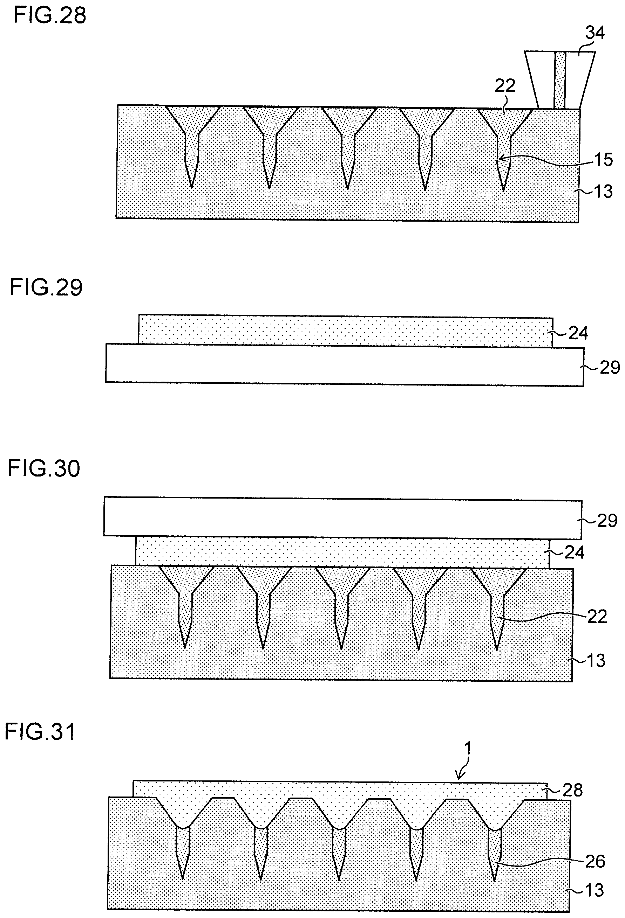

Next, a third aspect is described with reference to Figs. 28 to 31. As illustrated in Fig. 28, the drug-containing solution 22 is filled in the needle-like recessed portions 15 of the mold 13. As the filling method, similarly to the first aspect and the second aspect, filling may be performed in the state of bringing the distal end portion of the nozzle or the blade into contact with the mold 13. Then, as illustrated in Fig. 29, the non-drug-containing solution 24 is applied on a separate support (another support) 29. As a material of the support 29 , for example, polyethylene, polyethylene terephthalate, polycarbonate, polypropylene, an acrylic resin, and triacetylcellulose or the like may be used; however, the material is not limited to them. Next, as illustrated in Fig. 30, the non-drug-containing solution 24 formed on the support 29 is piled up (overlaid) on the mold 13 in which the drug-containing solution 22 has been filled in the needle-like recessed portions 15. Then, as illustrated in Fig. 31, by drying and curing the drug-containing solution 22 and the non-drug-containing solution 24, the polymer sheet 1 composed of the drug-containing layer 26 and the non-drug-containing layer 28 is formed.

In formation of the polymer sheet 1, a drying and curing process is a process of curing the drug-containing solution 22 inside the needle-like recessed portions 15 and/or the non-drug-containing solution 24 by drying the drug-containing solution 22 containing the solvent and/or the non-drug-containing solution 24.

After forming the polymer sheet 1 with the needle-like protruding portions formed on the front surface, in which the needle-like protruding portions are composed of the drug-containing layer 26 made of the drug-containing solution 22 and the non-drug-containing layer 28 made of the non-drug-containing solution 24, the process advances to a release process of releasing the polymer sheet 1 from the mold 13.

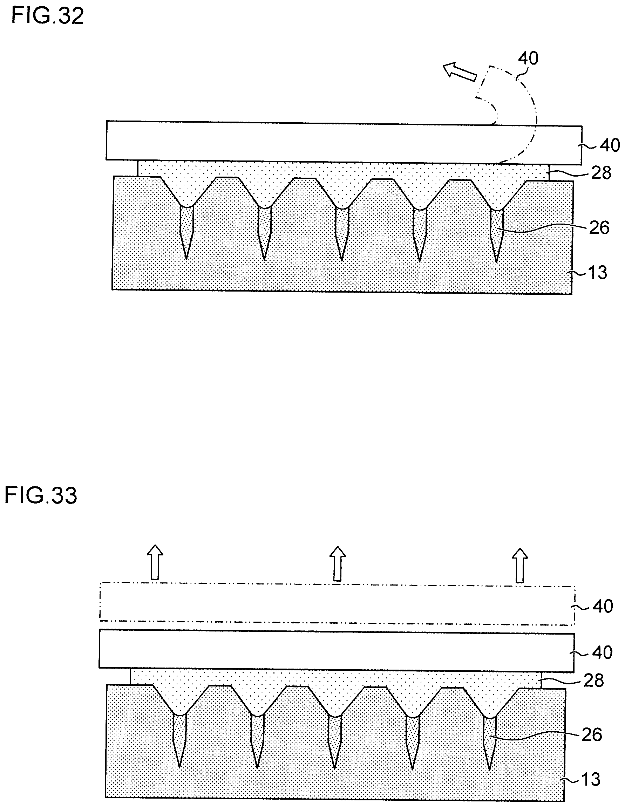

The method of releasing the polymer sheet 1 from the mold 13 is not limited in particular. Upon releasing, it is desired that the needle-like protruding portions do not bend or get broken. Specifically, as illustrated in Fig. 32, a sheet-like release plate 40 where an adhesive adhesion layer is formed is stuck onto the polymer sheet 1, and then release can be performed in a manner such that the release plate 40 is peeled off from an end portion. However, by that method, there is a possibility that the needle-like protruding portions bend. Therefore, as illustrated in Fig. 33, the following method can be adopted in which a sucker (not shown in the figure) is placed on the polymer sheet 1, and then the release plate 40 is vertically pulling up while sucking it by the air can be applied.

Normally, in the case of releasing a structure having the needle-like protruding portions of the high aspect ratio from the mold 13, since a contact area is large, strong stress is applied. It is concerned that the fine needle which is the needle-like protruding portion is destroyed and remains inside the needle-like recessed portion 15 without being released from the mold 13 and the fabricated transdermal absorption sheet has a defect.

Therefore, the mold 13 is composed of a material in which the Young's modulus is increased on the front surface side (the side where the transdermal absorption material is filled) of the mold and the Young's modulus is decreased on the back surface side (the distal end side of the needle-like protruding portions of the transdermal absorption sheet) of the mold. Thereby, it is possible to mitigate the stress applied to the needle-like protruding portions of the transdermal absorption sheet when releasing the mold.

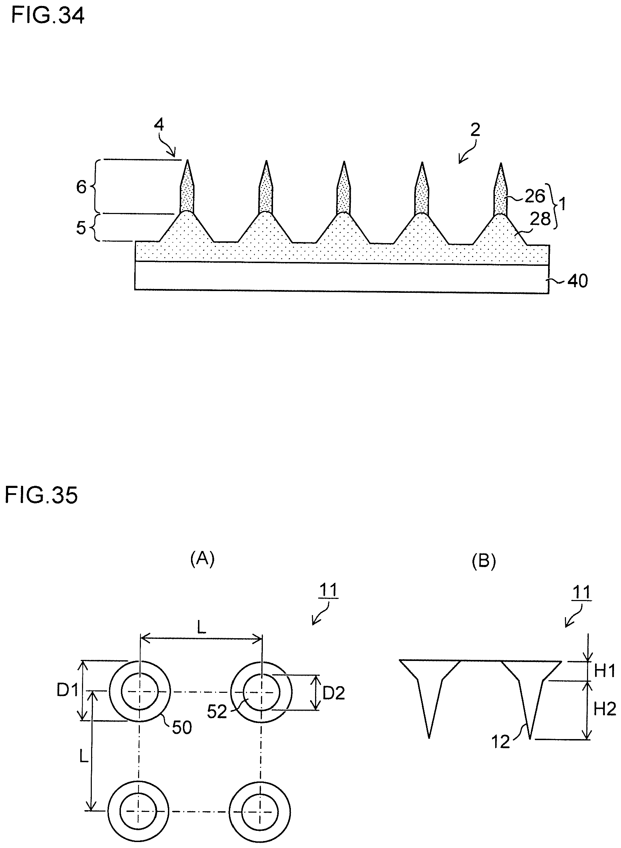

Fig. 34 illustrates a transdermal absorption sheet 2 which is composed of the polymer sheet 1 released from the mold 13. The transdermal absorption sheet 2 includes the release plate 40, the drug-containing layer 26 formed on the release plate 40, and the layer 28 practically not containing the drug. Each needle-like protruding portion 4 of the transdermal absorption sheet 2 is configured by a conical base portion 5 and a needle portion 6 on the conical base portion 5, and the needle portion 6 mainly includes a needle portion having a conical shape or the pyramid shape and a body portion having a columnar shape or a rectangular columnar shape. However, the shape of the needle-like protruding portion 4 is not limited thereto.

### [Example]

Hereinafter, the present invention is further specifically described with an example of the present invention. Note that a material, a use amount, a ratio, processing contents and a processing procedure or the like indicated in the following example can be appropriately changed without deviating from the gist of the present invention. Therefore, the scope of the present invention should not be exclusively interpreted by the specific example indicated below.

### (Fabrication of mold)

The original plate 11 was fabricated by machining so that the needle-like structure portions 12 each having a needle-like structure in the two-dimensional array of 10 columns × 10 rows at a pitch L of 1000 µm, on a front surface of a smooth Ni plate with a side length of 40 mm. The needle-like structure has a shape in which a cone 52 with a diameter D2 of 300 µm and a height H2 of 500 µm is formed on a truncated cone 50 with a diameter D1 of 500 µm at the bottom surface and a height HI of 150 µm, as illustrated in Part (A) and Part (B) in Fig. 35.

The materials the composite film (multilayer) includes: various kinds of silicone resin (SILASTIC MDX4-4210 made by Dow Corning Corp., MED-6019, MED-6015, MED-6010 made by Nusil Technology LLC); and the material prepared by surface-modifying zirconia particles using a silane coupling agent (Z-6040, Z-6043, or Z-6011, made by Dow Corning Corp.) in a water and methanol mixed solution containing 1 mass% of the silane coupling agent, filtrating the resultant, drying and comminuting the surface modified zirconia particles obtained by the filtration, and dispersing the surface modified zirconia particles in the silicone resin. Then, the composite film was formed from the prepared materials on the original plate 11 having the needle-like structure portions 12. The composite film was thermally cured for five hours at 80°C to 200°C, and released from the original plate 11. Thereby an inverse article of the needle-like structure portions 12 was fabricated. An outer side of a planar portion, where the needle-like recessed portions two-dimensionally arrayed in 10 columns × 10 rows were formed at a center part, was cut off from the inverse article so that each side of the inverse article had a length of 30 mm, and the trimmed inverse article was used as the mold. The side where the opening of each needle-like recessed portion was wide was defined as the front surface of the mold and the surface including the through-holes (air vent holes) with the diameter of 30 µm was defined as the back surface of the mold.

The mold with a rigidity distribution in the thickness direction was fabricated in the following manner. First, a film having a film thickness 40 µm and the Young's modulus which was greater than SILASTIC MDX4-4210 made by Dow Corning Corp. was fabricated on the original plate 11 as a mold front surface layer, and then, a film using SILASTIC MDX4-4210 made by Dow Corning Corp. was laminated as a mold back surface layer. The Young's modulus of the surface layer of the mold was increased by: adding MED-6019, MED-6015, MED-6010 made by Nusil Technology LLC, or a mixture of the three, or the mixture of the three after addition of the zirconia particles surface-modified by the silane coupling agent made by Dow Corning Corp. further to the mixture of the three; and adjusting a curing temperature and curing time.

### (Preparation of drug-containing solution)

The drug-containing solution was prepared in the following manner. Hydroxyethyl starch (made by Fresenius Kabi AG) was dissolved with water and prepared into an aqueous solution of 8%. Then, to the resultant solution, 2 mass% of human serum albumin (made by Wako Pure Chemical Industries, Ltd.) and 0.7 mass% of Evans blue pigments (made by Wako Pure Chemical Industries, Ltd.) were added as the drug.

### (Formation of drug-containing layer)

A gas permeable film (NTF-8031 made by NITTO DENKO CORPORATION) with a side length of 15 mm was placed on a horizontal vacuum base, and the mold was placed on it such that the front surface is turned up. The gas permeable film and the mold were fixed to the vacuum base by sucking them from a mold back surface direction with a suction pressure of 50 kPa. A nozzle made of the stainless steel having a shape as illustrated in Fig. 13 was prepared. At the center of the lip portion having a length of 20 mm and a width of 2 mm, the slip shaped opening having a length of 12 mm and a width of 0.2 mm was formed. The nozzle was attached to a syringe. Inside the syringe and the nozzle, 3 mL of the drug-containing solution was loaded. The nozzle was adjusted such that the opening was parallel with the first column configured by the plurality of needle-like recessed portions formed on the front surface of the mold. At a position at an interval of 2 mm from the first column in a direction opposite to the second column, the nozzle was pressed to the mold with the pressure of 1.37 × 10⁻² N/mm². While moving the nozzle in the direction parallel to the length direction of the opening at 10 mm/sec with the nozzle being kept pressed, the drug-containing solution was discharged from the opening for 10 seconds at 0.31 µL/sec by a dispenser. The movement of the nozzle was stopped at a position at the interval of 2 mm from the tenth column of the plurality of two-dimensionally arrayed needle-like recessed portions, in the direction opposite to the ninth column, and the nozzle was separated from the mold. The drug-containing solution adhered in an area apart, by 1 mm or more, from the outer periphery of the plurality of two-dimensionally arrayed needle-like recessed portions, was removed. Then, the resultant was dried for 30 minutes at 30°C and 40 RH% inside a thermohygrostat bath to form the drug-containing layer. After drying, by imparting a tape with low adhesive power to the front surface of the mold and peeling the tape off from the mold, all the drug-containing layer stuck to the part other than the needle-like recessed portions of the mold was removed.

### (Measurement of drug content)

Each of the mold and the tape with the low adhesive power was immersed in 1 mL of water inside a container with a lid of 5 mL, the lid of the container was loosened, pressurization was performed for 10 minutes at 0.5 MPa inside a pressurizing deaerator, then the container was tightly closed and ultrasonic cleaning was executed for 30 minutes. After confirming that there was no pigment remaining on the mold and the tape with the low adhesive power, for each solution, absorbance of a wavelength 620 nm was measured by a microplate absorbance reader (Sunrise series of Tecan Group Ltd.), and the content of the drug-containing layer inside the needle-like recessed portions and outside the needle-like recessed portions of the mold was calculated.

The mold was fabricated so as to have a Young's modulus varying in the range from 1.5 MPa to 501.6 MPa as illustrated in Table 1. A single mold was fabricated under the same condition separately from the mold to perform filling, and the Young's modulus of the mold was measured and calculated from the load when displacement of 40 µm was given to the mold, in the compression test for the side part of the needle-like recessed portions of the mold before filling the transdermal absorption material, using the micro-hardness meter HM2000 made by FISCHER INSTRUMENTS K.K. and the Berkovich indenter. For each mold, the transdermal absorption sheet was manufactured for five times, the filling amount was measured for each time, and the variation of the filling amount was evaluated.

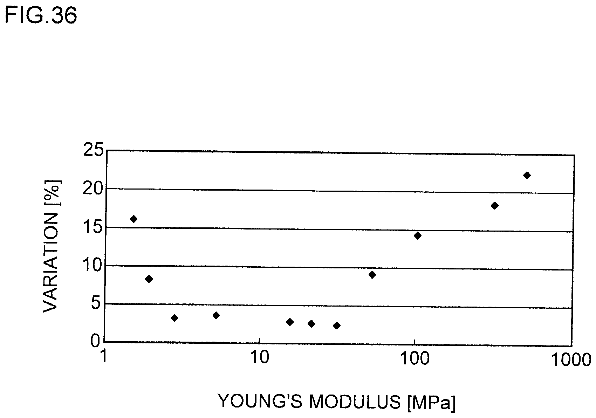

A result is illustrated in Table 1 and Fig. 36. Here, the variation of the filling amount was calculated in the following manner. A difference between a maximum filling amount and a minimum filling amount was obtained, then, the difference was divided by an average value of the filling amounts in five filling processes, and further divided by 2.

In contrast with the Young's modulus of 1.5 MPa indicated in comparative example 1, in the mold with the Young's modulus of 1.9 MPa or higher indicated in example 2, the variation of filling amount was improved, and the variation was 15% or lower. In addition, in the mold with the Young's modulus exceeding 500 MPa, the solution was stuck even to a flat part other than the needle-like recessed portions of the mold when filling the drug-containing solution. The filling amount was varied due to the sticking, and the filling accuracy was deteriorated.

**[Table 1]**

| | Young's modulus [MPa] | Variation of filling amount [%] |
|---|---|---|
| Comparative example 1 | 1.5 | 16.1 |
| Example 1 | 1.9 | 8.3 |
| Example 2 | 2.8 | 3.2 |
| Example 3 | 5.2 | 3.6 |
| Example 4 | 15.5 | 2.8 |
| Example 5 | 21.1 | 2.6 |
| Example 6 | 30.8 | 2.4 |
| Example 7 | 51.6 | 9.1 |
| Comparative example 2 | 102.1 | 14.3 |
| Comparative example 3 | 314.7 | 18.3 |
| Comparative example 4 | 501.6 | 22.3 |

When the solution is filled in the mold while scanning the nozzle in a state where the nozzle is not pushed into the mold in a thickness direction of the mold by a length equivalent to a roughness on the surface of the mold, it is conceivable that the solution jets out through a gap due to the surface roughness and the filling accuracy is deteriorated. In the mold with the Young's modulus exceeding 500 MPa, since the mold is too hard, it is conceivable that the nozzle cannot be sufficiently pushed in, the solution flows through the gap and the filling accuracy is deteriorated.

On the other hand, as illustrated in the comparative example 1, even when the Young's modulus is too low, the filling accuracy is deteriorated due to a crush amount of the needle-like recessed portions.

From the result of the examples described above, by setting the average value of the Young's modulus at the part within 40 µm from the front surface of the mold to be 1.9 MPa or higher and 100 MPa or lower, the variation of the filling amount to the needle-like recessed portions can be suppressed to 15% or lower, and the transdermal absorption sheet having the needle-like protruding portions can be accurately manufactured.

## Claims

1. A manufacturing method of a sheet having needle-like protruding portions (4), comprising:
a device preparation step of preparing a mold (13) having needle-like recessed portions (15) formed in a front surface of the mold (13), and a solution supply device (36) having a slit-like opening formed at a nozzle distal end portion;
a filling step of supplying a solution (22) from the solution supply device (36) to the mold (13) in a state that the nozzle distal end portion is pressed against the front surface of the mold (13) in a thickness direction thereof, and filling the solution (22) in the needle-like recessed portions (15); and
a moving step of moving the solution supply device (36) relatively to the mold (13) in a state that the nozzle distal end portion is brought into contact with the front surface of the mold (13),
wherein, as a hardness distribution in the thickness direction of the mold (13), an average value of a Young's modulus at a part within 40 µm from the front surface of the mold (13) is 1.9 MPa or higher and 100 MPa or lower, and an average value of a Young's modulus at a part over 40 µm from the front surface of the mold (13) is lower than the average value of the Young's modulus at the part within 40 µm from the front surface of the mold (13).

2. A manufacturing method of a sheet having needle-like protruding portions (4) comprising:
a device preparation step of preparing a mold (13) having needle-like recessed portions (15) formed in a front surface of the mold (13), and a solution supply device (136) having a blade (138);
a solution supply step of supplying a solution (22) from the solution supply device (136) to the a front surface of the mold (13); and
a moving step of moving the blade (138) relatively to the mold (13) in a state that the blade (138) is pressed against the front surface of the mold (13) in a thickness direction thereof,
wherein, as a hardness distribution in the thickness direction of the mold (13), an average value of a Young's modulus at a part within 40 µm from the front surface of the mold (13) is 1.9 MPa or higher and 100 MPa or lower, and an average value of a Young's modulus at a part over 40 µm from the front surface of the mold (13) is lower than the average value of the Young's modulus at the part within 40 µm from the front surface of the mold (13).

3. The manufacturing method of the sheet having the needle-like protruding portions (4) according to claim 1 or 2,
wherein an average value of a Young's modulus in an entire thickness direction of the mold (13) is 1.9 MPa or higher and 100 MPa or lower.

4. The manufacturing method of the sheet having the needle-like protruding portions (4) according to any one of claims 1 to 3,
wherein the mold (13) is formed of silicone resin.

## Patentansprüche

1. Fertigungsverfahren für ein Flachstück mit nadelartigen Vorsprüngen (4), umfassend:
einen Einrichtungsvorbereitungsschritt des Vorbereitens einer Form (13) mit nadelartigen Vertiefungen (15) in einer Frontfläche der Form (13), und einer Lösungszuführeinrichtung (36) mit einer schlitzartigen Öffnung an einem distalen Düsen-Endbereich;
einen Füllschritt des Zuführens einer Lösung (22) von der Lösungszuführeinrichtung (36) zu der Form (13) in einem Zustand, in welchem der distale Düsen-Endbereich gegen die Frontfläche der Form (13) in deren Dickenrichtung gepresst wird, und des Füllens der Lösung (22) in die nadelartigen Vertiefungen (15); und
einen Bewegungsschritt des Bewegens der Lösungszuführeinrichtung (36) relativ zu der Form (13) in einem Zustand, in welchem der distale Düsen-Endbereich in Berührung mit der Frontfläche der Form (13) gebracht ist,
wobei als eine Härteverteilung in der Dickenrichtung der Form (13) ein durchschnittlicher Wert eines Young-Modul in einem Bereich innerhalb von 40 µm gegenüber der Frontfläche der Form (13) 1,9 MPa oder mehr und 100 MPa oder weniger beträgt, und ein durchschnittlicher Wert eines Young-Moduls in einem Bereich oberhalb von 40 µm gegenüber der Frontfläche der Form (13) geringer ist als der durchschnittliche Wert des Young-Moduls in dem Bereich innerhalb von 40 µm gegenüber der Frontfläche der Form (13).

2. Fertigungsverfahren für ein Flachstück mit nadelähnlichen Vorsprüngen, umfassend:
einen Einrichtungsvorbereitungsschritt des Vorbereitens einer Form (13) mit nadelähnlichen Vertiefungen (15) in einer Frontfläche der Form (13), und einer Lösungszuführeinrichtung (136) mit einer Rakel (138);
einen Lösungszuführschritt des Zuführens einer Lösung (22) aus der Lösungszuführeinrichtung (136) zu einer Frontfläche der Form (13); und
einen Bewegungsschritt des Bewegens der Rakel (138) relativ zu der Form (13) in einem Zustand, in welchem die Rakel (138) gegen die Frontfläche der Form (13) in deren Dickenrichtung gepresst wird,
wobei als eine Härteverteilung in der Dickenrichtung der Form (13) ein durchschnittlicher Wert eines Young-Modul in einem Bereich innerhalb von 40 µm gegenüber der Frontfläche der Form (13) 1,9 MPa oder mehr und 100 MPa oder weniger beträgt, und ein durchschnittlicher Wert eines Young-Moduls in einem Bereich oberhalb von 40 µm gegenüber der Frontfläche der Form (13) geringer ist als der durchschnittliche Wert des Young-Moduls in dem Bereich innerhalb von 40 µm gegenüber der Frontfläche der Form (13).

3. Verfahren nach Anspruch 1 oder 2,
bei dem ein durchschnittlicher Wert eines Young-Moduls in einer Gesamt-Dickenrichtung der Form (13) 1,9 MPa oder mehr und 100 MPa oder weniger beträgt.

4. Verfahren nach einem der Ansprüche 1 bis 3,
bei dem die Form (13) aus Silikonkautschuk gebildet ist.

## Revendications

1. Procédé de fabrication d'une feuille présentant des portions saillantes en forme d'aiguilles (4), comprenant :
une étape de préparation de dispositif, pour préparer un moule (13) présentant des portions en retrait en forme d'aiguilles (15) formées dans une surface avant du moule (13), et un dispositif d'alimentation en solution (36) comprenant une ouverture en forme de fente formée sur une portion d'extrémité distale de buse ;
une étape de remplissage, pour alimenter une solution (22) du dispositif d'alimentation en solution (36) vers le moule (13) dans un état où la portion d'extrémité distale de buse est pressée contre la surface avant du moule (13) dans une direction d'épaisseur de celui-ci, et pour remplir la solution (22) dans les portions en retrait en forme d'aiguilles (15), et
une étape de déplacement pour déplacer le dispositif d'alimentation en solution (36) de manière relative par rapport au moule (13) dans un état où la portion d'extrémité distale de buse est amenée en contact avec la surface avant du moule (13),
dans lequel, comme distribution de la dureté dans la direction d'épaisseur du moule (13), une valeur moyenne d'un module de Young au niveau d'une partie dans les 40 µm à partir de la surface avant du moule (13) est supérieure ou égale à 1,9 MPa et inférieure ou égale à 100 MPa, et une valeur moyenne d'un module de Young au niveau d'une partie au-delà de 40 µm à partir de la surface avant du moule (13) est inférieure à la valeur moyenne du module de Young au niveau de la partie dans les 40 µm à partir de la surface avant du moule (13).

2. Procédé de fabrication d'une feuille présentant des portions saillantes en forme d'aiguilles (4), comprenant :
une étape de préparation de dispositif, pour préparer un moule (13) présentant des portions en retrait en forme d'aiguilles (15) formées dans une surface avant du moule (13), et un dispositif d'alimentation en solution (136) présentant une lame (138) ;
une étape d'alimentation en solution, pour alimenter une solution (22) du dispositif d'alimentation en solution (136) vers la surface avant du moule (13), et
une étape de déplacement, pour déplacer la lame (138) de manière relative par rapport au moule (13) dans un état où la lame (138) est pressée contre la surface avant du moule (13) dans une direction d'épaisseur de celui-ci,
dans lequel, comme distribution de la dureté dans la direction d'épaisseur du moule (13), une valeur moyenne d'un module de Young au niveau d'une partie dans les 40 µm à partir de la surface avant du moule (13) est supérieure ou égale à 1,9 MPa et inférieure ou égale à 100 MPa, et une valeur moyenne d'un module de Young au niveau d'une partie au-delà de 40 µm à partir de la surface avant du moule (13) est inférieure à la valeur moyenne du module de Young au niveau de la partie dans les 40 µm à partir de la surface avant du moule (13).

3. Procédé de fabrication d'une feuille présentant des portions saillantes en forme d'aiguilles (4) selon la revendication 1 ou 2,
dans lequel une valeur moyenne d'un module de Young dans une direction d'épaisseur entière du moule (13) est supérieure ou égale à 1,9 MPa et inférieure ou égale à 100 MPa.

4. Procédé de fabrication d'une feuille présentant des portions saillantes en forme d'aiguilles (4) selon l'une quelconque des revendications 1 à 3,
dans lequel le moule (13) est formé en résine silicone.
